# EUROPEAN PATENT APPLICATION

(11) **EP 1 547 647 A1**
(43) Date of publication of application: **29.06.2005**
(21) Application number: 04256458.3
(22) Date of filing: 20.10.2004
(51) Int. Cl.: A61N 1/05, C23C 14/06, C22C 19/00, C22C 27/06, H01B 7/00, H01B 7/04

(54) **Implantable electrical lead wire**

(30) Priority: 20.10.2003 US 512741 P
(71) Applicant: Greatbatch-Hittman, Incorporated, Columbia, MD 21045 (US)
(72) Inventor: O'Brien, Robert C., Sykesville Maryland 21784 (US)
(74) Representative: Colmer, Stephen Gary

(57) **Abstract**

Implantable electrical lead wires, such as cobalt-chromium-molybdenum alloy wires, are coated with a metal, ceramic, or carbon to a thickness of about 100 nm or less to provide a non-reactive interface to polyurethane sheathing materials. Preferred is sputter coating an amorphous carbon intermediate the alloy wire and the polyurethane sheath.

## Description

### CROSS-REFERENCE TO RELATED APPLICATION

This application claims priority from provisional application Serial No. 60/512,741, filed October 20, 2003.

### BACKGROUND OF THE INVENTION

### 1. Field of the Invention

The present invention relates to implantable electrical lead wires such as those used in cardiac pacing and neurostimulation. More particularly, the present invention relates to a solution to the chronic compatibility problems of lead wires with the materials used to insulate them.

The primary requirements of lead wires are conductivity and fatigue resistance. Because lead wires are designed so that body fluids never come into contact with the conductor material, biocompatibility has only been considered a secondary requirement. However, with the introduction of polyurethane as an insulator for lead wires, it is now known that an interaction can be initiated at the conductor/insulator interface in which the insulator material is degraded by a metal ion oxidation mechanism. The ions are supplied by cobalt, chromium and molybdenum from the lead wires. Providing a thin film layer of inert material on the lead wires intermediate the polyurethane coating prevents this.

### 2. Prior Art

Pacing lead wires are typically manufactured from alloys such as stainless steels, ELGILOY® alloy, MP35N® alloy, and DBS/MP. DBS is a drawn-brazed-strand having a silver core surrounded by strands of stainless steel or MP35N® alloy. These alloys have particularly advantageous mechanical and electrical properties which when coiled allow them to display appropriate mechanical and electrical characteristics for use in electrical stimulation leads. However, MP35N®, ELGILOY® and DBS/MP all include cobalt, molybdenum and chromium as significant constituents. It is now known that cobalt, chromium, and molybdenum accelerate oxidative degradation of the polyurethane sheathing used in pacing leads. To a lesser degree, it appears that stainless steel also accelerates polyurethane degradation.

For that reason, the introduction of polyurethane as a biocompatible insulator has led to efforts to passivate the coil/insulator interface by choosing a non-reactive conductor material for the coil. The disadvantage is that the very desirable fatigue resistance of nickel, cobalt, chromium, and molybdenum materials and their alloys is lost.

A more effective approach has been to coat the wires with a non-interacting material, such as titanium or platinum. This is described in U.S. Patent No. 5,040,544 to Lessar et al. The disadvantage of using titanium and platinum as coating materials, however, is that they can be damaged during the coating process and lead assembly, as well as by the stylet during implantation. Another disadvantage is their generally poor adhesion to the wire throughout the deformation process during coiling to low diameter coils. Leads are coiled so they can withstand constant flexing and bending forces as a result of body movement.

The deformation process can also result in the development of small breaches or cracks in the titanium and platinum coating. Lessar et al. do not necessarily see this as a significant problem when they state "simply covering a high percentage of the surface area of the conductor provides substantial improvement in resistance to oxidative degradation of the polyurethane sheath. Moreover, the inventors have determined that actual physical contact between the conductor and the polyurethane insulation is a significant factor in the oxidative degradation of the polyurethane insulation. Even in the absence of an insulative outer layer, the typical cracks and breaches in the sputtered coating due to winding are unlikely to produce significant areas of contact between the base metal of the coil and the polyurethane insulation."

Nonetheless, it is desirable to provide a continuous inert coating between the lead wire material and the polyurethane sheath that readily adheres to the lead wire and is totally free of cracks and breaches. Any degree of compromise in the insulation layer is cause for concern.

### SUMMARY OF THE INVENTION

Polyurethane insulator degradation is prevented by means of a barrier coating consisting of a very thin sputtered film of selected metal, ceramic, and carbon in the form of amorphous carbon, turbostratic carbon, diamond-like carbon, and the like. These films are characterized by very good hardness, durability, and adhesion. When applied as a thin film, they readily conform to the lead wire metal surface as the wire is formed into a coil. At very thin film thicknesses, the films readily adapt to the stresses of coiling a wire into a helical shape to provide an effective barrier layer.

These and other aspects of the present invention will become more apparent to those skilled in the art by reference to the following description and to the appended drawings.

### BRIEF DESCRIPTION OF THE DRAWINGS

FIG. 1 is a perspective view, partly in phantom, showing an implantable medical device 10 connected to a pair of electrodes 20 and 22 by respective coiled leads 16 and 18.
FIG. 2 is a cross-sectional view along line 2-2 of FIG. 1.
FIG. 3 is a photograph at 200x magnification of a 0.004-inch diameter MP35N® wire coiled to a final diameter of about 0.025 inches.
FIG. 4 is a schematic diagram of a sputtering chamber used in the direct sputtering of a protective coating on a wire according to the present invention.
FIG. 5 is a photograph at 5,000x magnification of a 200 nm titanium film with adhesion failure.
FIG. 6 is a photograph at 5,000x magnification of a titanium film stopped short of complete island coalescence with the film remaining adhered through the coiling process.
FIG. 7 is a photograph at 500x magnification of a coil made of a 0.004-inch diameter MP35N® wire that was sputter coated with titanium and then coiled to a final diameter of about 0.25 inches.

### DETAILED DESCRIPTION OF THE PREFRRED EMBODIMENTS

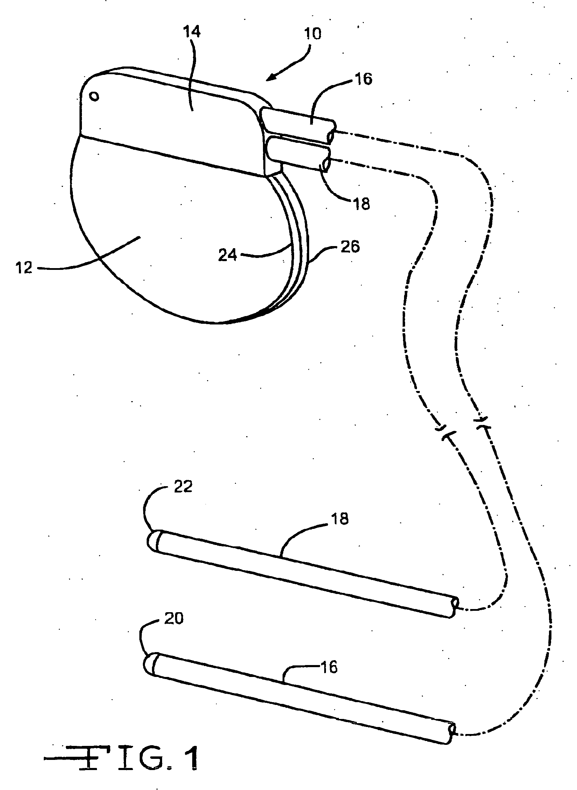

Referring now to the drawings, FIG. 1 illustrates an implantable medical device 10 comprising a housing 12 supporting a header 14 connecting leads 16 and 18 to respective electrodes 20 and 22. The housing 12 is of a conductive material, such as of titanium or stainless steel. Preferably, the medical device housing 12 comprises mating clamshell portions 24 and 26 in an overlapping relationship. The clamshell housing portions are hermetically sealed together, such as by laser or resistance welding, to provide an enclosure for control circuitry (not shown) connected to a power supply (not shown), such as a battery. There may also be a capacitor for a medical device such as a defibrillator. U.S. Patent No. 6,613,474 to Frustaci et al. contains a more detailed description of a housing comprising mating clamshell portions. This patent is assigned to the assignee of the present invention and incorporated herein by reference. The housing 12 can also be of a deep drawn, prismatic and cylindrical design, as is well known to those skilled in the art.

The header 14 is mounted on the housing 12 and comprises a body of molded polymeric material supporting terminal blocks (not shown) that provide for plugging the proximal ends of leads 16 and 18 therein to electrically connect them to the control circuitry and power supply contained inside the housing. The electrodes 20 and 22 are located at the distal ends of the respective leads 16, 18. For a more detailed description of the header assembly, reference is made to U.S. Application Serial No. 10/701,849, filed November 5, 2003, which is assigned to the assignee of the present invention and incorporated herein by reference.

The electrodes 20, 22 are surgically secured to body tissue whose proper functioning is assisted by the medical device. In that respect, the implantable medical device 10 is exemplary of any one of a number of known therapeutic devices such as an implantable cardiac pacemaker, a defibrillator, and the like. In such devices, therapy is in the form of an electrical pulse delivered to the body tissue, such as the heart, by means of implanted electrodes such as those shown in FIG. 1.

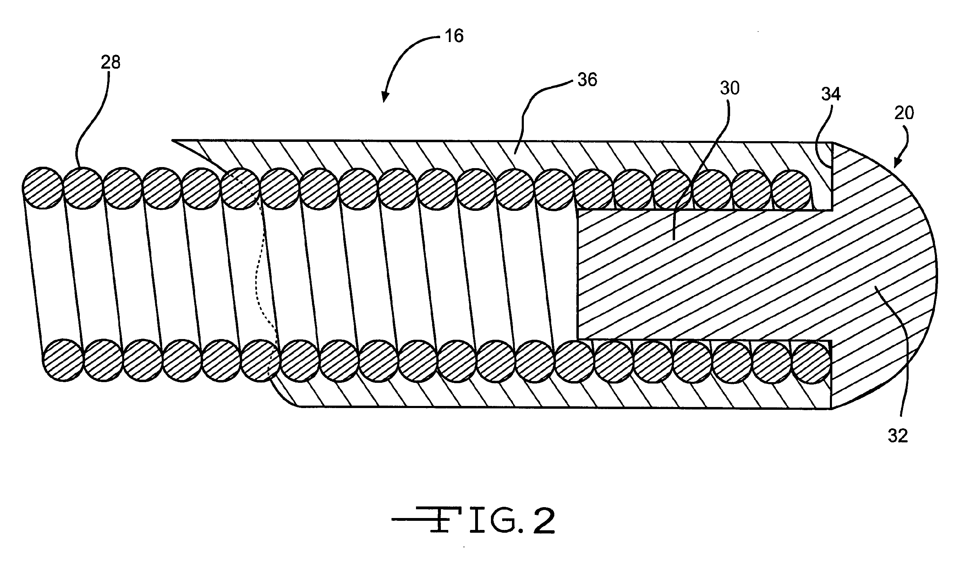

The electrodes 20, 22 are similar in construction, although that is not necessary. Nonetheless, the present invention will be described with respect to electrode 20 shown in greater detail in FIG. 2 for the sake of simplicity. As shown, electrode 20 is connected to the medical device 10 by a helical strand or filar 28 comprising the lead 16. The electrode 20 comprises a cylindrically shaped proximal shaft 30 supporting a head 32 having a radiused hemispherical shape. A step 34 is between the shaft and the head. The diameter of the proximal shaft 30 is slightly larger than the inside diameter of the helical strand 28 so the electrode 20 stays in place inside the coil while it is being assembled and during use. Suitable materials for the electrode 20 include carbon such as pyrolytic carbon, titanium, zirconium, niobium, molybdenum, palladium, hafnium, tantalum, tungsten, iridium, platinum, gold, and alloys thereof.

As shown in the final assembly of FIG. 2, encasing the helical strand 28 up to the step 34 in a biocompatible elastomeric material 36, such as silicone or polyurethane, completes the electrode. Only the active'surface of the head 32 is left exposed. This surface may be impregnated with liquid silicone or other biocompatible resin that is then polymerized to seal the porosity, and to keep body fluids from infusing into the porous electrode and reaching the helical strand 28. The remaining surfaces of the electrode 20 received in the helical strand 28 and exposed to the elastomeric material 36 are preferably roughened by grit blasting, machining marks, knurling, and the like to improve adhesion thereto.

Implantable leads are made of wires typically about 0.002 to 0.005 inches in diameter formed into coils or helical strands about 0.015 inches to about 0.030 inches in diameter. As previously discussed, conductive, fatigue resistant materials such as stainless steel, ELGILOY®, MP35N®, and DBS/MP alloys are preferred for the helical strand 28. These materials exhibit the desired mechanical properties of low electrical resistance, corrosion resistance, flexibility and strength required for long term duty inside a human body, and the like.

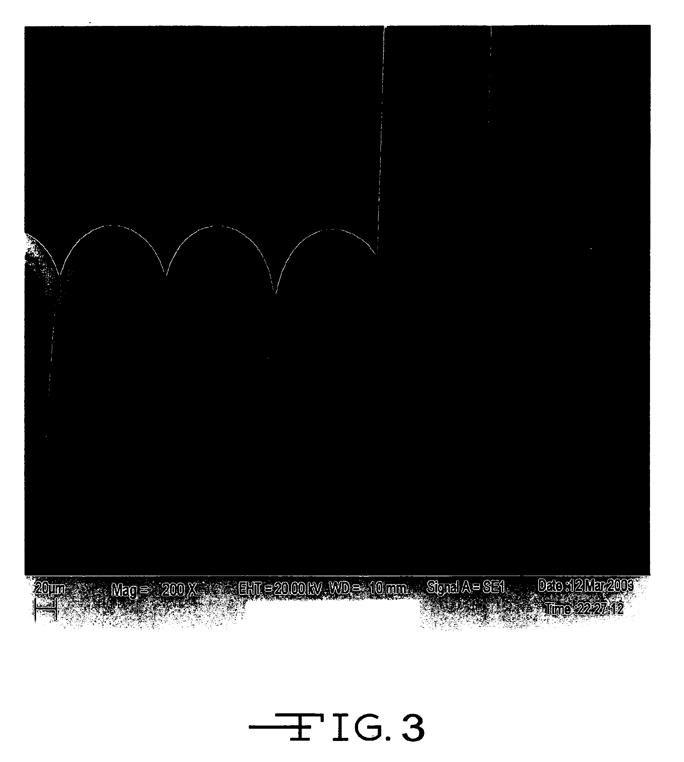

The photograph in FIG. 3 shows a 0.004-inch diameter MP35N® wire at a magnification of 200x coiled to a final diameter of about 0.025 inches. On the right side, a portion of the wire is shown prior to being coiled. On the left side of the photograph, it can be seen that during the coiling process the material at the outside diameter surface of the wire has undergone plastic deformation. This is typically up to about 25 percent over its unstrained length. Plastic strain is dimensionless, having the units of length/length. The 25% number refers to a plastic extension over unstrained length of 0.25 inch per inch. Grain rotation, grain boundary slip, and slip bands within grains create an "orange peel" surface texture on the wire to which the coating must conform in order to be an effective barrier.

The problem is that cobalt, chromium, and molybdenum comprising ELGILOY® (cobalt 40%, chromium 20%, nickel 15%, molybdenum 7%, manganese 2%, carbon < 0.10%, beryllium < 0.10%, and iron 5.8%, by weight), MP35N® (nickel 35%, cobalt 35%, chromium 20%, and molybdenum 10%, by weight), and DBS/MP alloys react with elastomeric materials used to protect them from body fluids, especially urethanes, with the result that the elastomer is degraded and rendered at least partially ineffective. According to the present invention, a thin film layer of metal, ceramic, or carbon in the form of amorphous carbon, turbostratic carbon, diamond-like carbon is coated on the wire to prevent direct contact between the materials of the helical strand 28 and the elastomeric material 36 to help prevent this degradation. Preferably, the metal, ceramic or carbon coating is provided on the wire by a sputtering process.

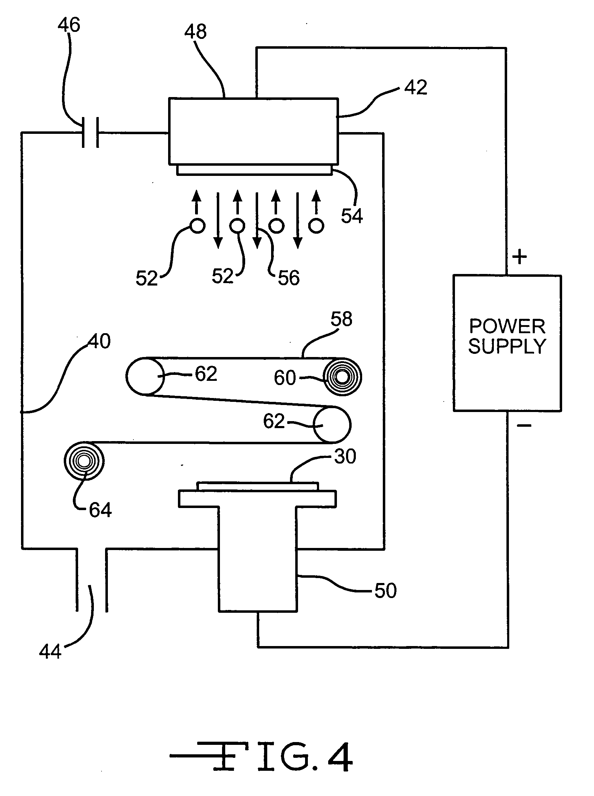

A schematic for a direct sputtering process of a metal, ceramic or carbon is shown in FIG. 4. The sputtering takes place in a stainless steel chamber 40. Sputtering guns 42, which are generally located at the top of the chamber 40, accomplish the actual sputtering function. The sputtering guns 42 are capable of movement in both the horizontal and vertical directions as desired.

The sputtering process begins by evacuating the chamber 40 of ambient air through evacuation port 44. An inert gas such as argon is then fed into the chamber 40 through a gas port 46. The argon gas is ionized using the cathode 48 and the anode 50 to generate an ion flux 52 that strikes a metal, ceramic or carbon target 54. The impact of the ion flux 52 ejects a sputtered flux 56 that travels and adheres to the wire substrate 58. The wire 58 is wound on a feeder spool 60 and fed by means of multiple-sheave pulleys 62 to a take-up reel 64 for several back-and-forth passes in front of the sputter cathode 48 with the target 54. Looping of the wire 58 around the pulleys 62 allows for higher wire feed rates, as well as assuring that all sides of the wire 58 are exposed to the sputter flux at some time during processing.

It is important to understand that sputtering is a momentum transfer process. Constituent atoms of the coating material are ejected from the surface of the target 54 because of momentum exchange associated with bombardment by energetic particles. The bombarding species are generally ions of heavy inert gas, usually argon. The flux 56 of sputtered atoms may collide repeatedly with the working gas atoms before reaching the wire substrate 58 where they condense to form the desired coating thereon.

Sputtering times vary depending on the coating material. However, experimentally it has been determined that sputtering times are about 1 to 5 minutes to generate a coating up to about 100 nm thick on the base wire 58. Generally, it has been found that the sputtering process applies the sputtered flux 56 as a coating according to a linear function, so the application time is easily adjusted accordingly to obtain the desired thickness.

For example, in the case of amorphous carbon, the coating is provided at thicknesses of about 10 nanometers (nm) to 50 nm before the wire is coiled into the helix shape. The 10 nm thick carbon coating thus corresponds to a deposition rate of approximately 1 angstrom being added every second. Amorphous carbon coatings about 10 nm to about 50 nm thick provide a completely non-reactive interface to polyurethane insulating materials while conforming to surface irregularities that occur during the coiling process. Regardless the coating material, coating thicknesses are about 100 nm, or less.

In addition to a carbon coating, it is contemplated that other thin film materials suitable for the coatings include any metal or ceramic that can be applied in a film sufficiently thin to allow it to adhere to a wire substrate through the coiling process and its associated plastic deformation. These include titanium, platinum, iridium, tantalum, palladium, niobium, gold, and alloys of these metals, and ceramics such as titanium nitride, aluminum oxide, aluminum nitride, and the like.

In that respect, thin films that are effective in the current invention are those that can be grown by a mechanism of 3D island growth, as described in Chapman, "Glow Discharge Processes" Wiley, 1980, 201-203. The film must be grown until the island coalescence phase of growth is almost complete, in order to maximize coverage of the substrate by the coating material. However, the film growth must be stopped before completion of the island coalescence phase, when the islands adhere to the substrate, but not to each other. Plastic strain of the wire during coiling increases the distance between islands, but does not result in separation of the growing film from the wire substrate. In effect, atoms of the coating material bond together to act as a unit or island with respect to plastic deformation of the substrate so that when the substrate is deformed, the islands move with the substrate. Suitable coating thicknesses are about 100 nm or less.

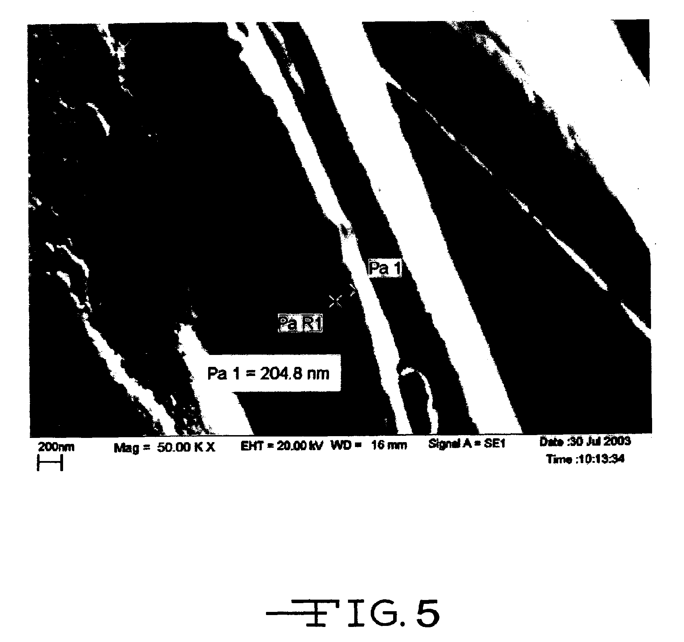

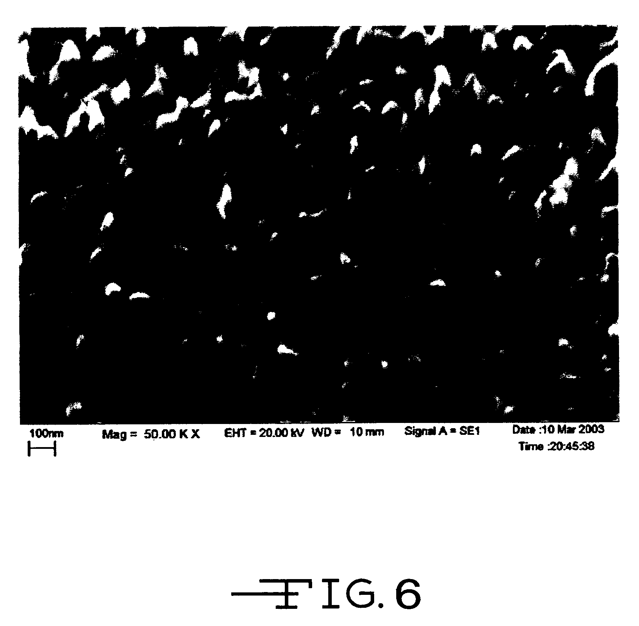

Experiments have shown that a titanium film with a thickness of about 200 nm (FIG. 5) has already passed the coalescence phase and is subject to adhesion failure due to the coalesced islands responding to the strain as large continuous units, rather than as individual islands. However, films in which the islands have not completed coalescence do not undergo adhesion failure on plastic strain because the islands are not attached to each other. Titanium coated to a thickness of about 100 nm in which the island coalescence process is just short of completion is shown in FIG. 6. In this photograph, the average island diameter is approximately on the same order as the coating thickness, that is, about 50 nm to about 100 nm.

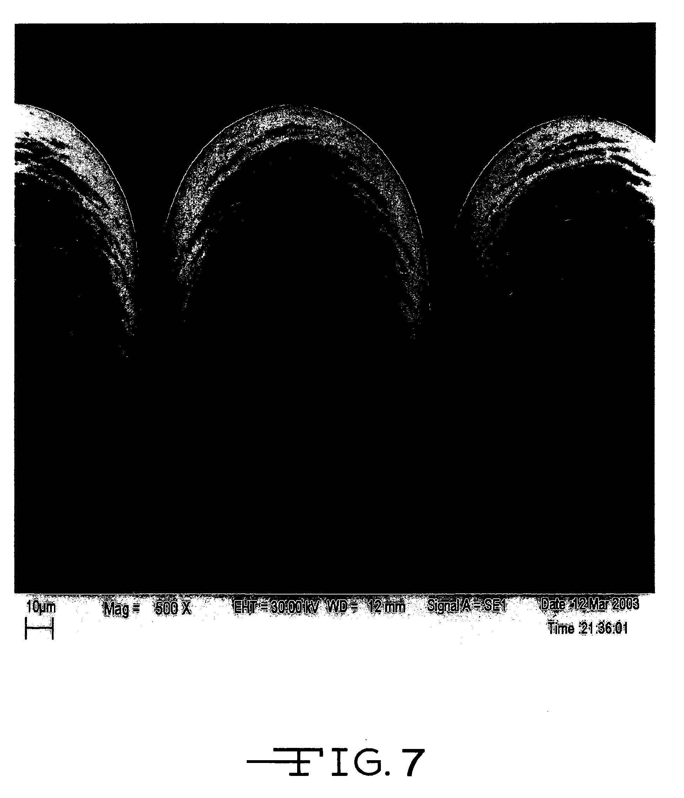

FIG. 7 is a photograph showing a 0.004-inch diameter MP35N® wire at a magnification of 500x coiled to a final diameter of about 0.025 inches. This wire is provided with a sputter coated titanium coating according to the present invention. It can be seen that the titanium readily conforms to the lead wire metal surface and stays adhered thereto even after the wire has been formed into a helical coil.

Other thin film deposition processes useful with the invention include thermal spraying processes such as chemical combustion spraying processes and electric heat spraying processes. Chemical combustion spraying processes include powder flame spraying, wire/rod flame spraying, high velocity oxygen fuel flame spraying and detonation/explosive flame spraying. Electrical heat spraying processes include electric arc or twin-wire arc spraying and plasma spraying. These spraying processes are generally delineated by the methods used to generate heat to plasticize and/or atomize the coating material.

Powder flame spraying involves the use of a powder flame spray gun consisting of a high capacity, oxygen-fuel gas torch and a hopper containing the coating material in powder or particulate form. A small amount of oxygen from the gas supply is diverted to carry the powdered coating material by aspiration into the oxygen-fuel gas flame where the powder is heated and propelled by the exhaust flame onto the substrate. The fuel gas is usually acetylene or hydrogen and temperatures in the range of about 3,000°F, to 4,500°F. are typically obtained. Particle velocities are on the order of about 80 to 100 feet per second.

Wire/rod flame spraying utilizes a wire of the coating material. The wire is continuously fed into an oxy-acetylene flame where it is melted and atomized by an auxiliary stream of compressed air and then deposited as the coating on the substrate. This process also lends itself to use of plastic tubes filled with the coating material in a powder form.

High velocity, oxygen fuel flame spraying is a continuous combustion process that produces exit gas velocities estimated at about 4,000 to 5,000 feet per second and particle speeds of about 1,800 to 2,600 feet per second. This is accomplished by burning a fuel gas (usually propylene) with oxygen under high pressure (60 to 90 psi) in an internal combustion chamber. Hot exhaust gases are discharged from the combustion chamber through exhaust ports and thereafter expanded in an extending nozzle. The coating powder is fed axially into the extending nozzle and confined by the exhaust gas stream until the coating material exits in a thin high speed jet to produce coatings which are more dense than those produced by powder flame spraying.

A modified flame spraying process is referred to as a flame spray and fuse process. In this process, the coating active material is deposited onto the substrate using one of the above described flame-spraying processes followed by a fusing step. Fusing is accomplished by one of several techniques such as flame or torch, induction, or in vacuum, inert or hydrogen furnaces. Typical fusing temperatures are between 1,850°F. to 2,150°F., and in that respect, the substrate material needs to be able to withstand this temperature range.

In contrast to the previously described thermal spray processes, i.e., powder flame spraying, wire/rod flame spraying and high velocity, oxygen fuel flame spraying, which utilize the energy of a steady burning flame, the detonation/explosive flame spraying process uses detonation waves from repeated explosions of oxy-acetylene gas mixtures to accelerate the powered electrode active material. Particulate velocities on the order of 2,400 feet per second are achieved and the coatings are extremely strong, hard, dense and tightly bonded.

The electrical heating thermal spraying process, referred to as the twin-wire arc spraying process uses two consumable wires of electrode active material. The wires are initially insulated from each other and simultaneously advanced to meet at a focal point in an atomizing gas stream. Contact tips serve to precisely guide the wires and to provide good electrical contact between the moving wires and power cables. Heating is provided by means of a direct current potential difference applied across the wires to form an arc that melts the intersecting wires. A jet of gas (normally compressed air) shears off molten droplets of the melted electrode active material and propels this material onto the substrate. Sprayed coating material particle sizes can be changed with different atomizing heads and wire intersection angles. Direct current is supplied at potentials of about 18 to 40 volts; depending on the material to be sprayed; the size of particle spray increasing as the arc gap is lengthened with rise in voltage. Voltage is therefore maintained at a higher level consistent with arc stability to provide larger particles and a rough, porous coating. Because high arc temperatures (in excess of about 7,240°F.) are typically encountered, twin-wire arc sprayed coatings have high bond and cohesive strength.

Plasma spraying involves the passage of a gas or a gas mixture through a direct current arc maintained in a chamber between a coaxially aligned cathode and water-cooled anode. The arc is initiated with a high frequency discharge that partially ionizes the gas to create a plasma having temperatures that may exceed 30,000°F. The plasma flux exits the gun through a hole in the anode that acts as a nozzle and the temperature of the expelled plasma effluent falls rapidly with distance. Powdered coating material feedstock is introduced into the hot gaseous effluent at an appropriate point and propelled to the substrate by the high velocity stream. The heat content, temperature and velocity of the plasma gas are controlled by regulating arc current, gas flow rate, and the type and mixture ratio of gases and by the anode/cathode configuration.

Other thin film physical vapor deposition and chemical vapor deposition methods, including evaporation and laser ablation are also suitable deposition processes.

It should be pointed out that while the present invention has been described with respect to a coating on a coiled or helix wire, it should not be so limited. Instead, the coating can be on any deformable substrate such as a stent, stylet, or other device, whether intended for an implatable application, or not.

It is appreciated that various modifications to the inventive concepts described herein may be apparent to those of ordinary skill in the art without departing from the spirit and scope of the present invention as defined by the appended claims.

## Claims

1. A medical electrical lead, comprising;
a) an elongated insulative sheath of an elastomer; for example, polyurethane;
b) an elongated conductor at least partially housed within the polyurethane sheath;
c) an electrode coupled to a distal end of the elongated conductor;
d) an electrical connector coupled to a proximal end of the elongated conductor; and
e) wherein the elongated conductor is comprised of a wire of a first alloy including at least one of cobalt, molybdenum, and chromium provided with a coating of a biocompatible carbonaceous material that does not interact with the polyurethane sheath.

2. A medical electrical lead comprising:
a) a multifilar conductor;
b) an elongated insulative sheath of an elastomer; for example, polyurethane at least partially encasing the multifilar conductor;
c) at least one electrode coupled to a distal end of at least one of the filers in the multifilar conductor; and
d) wherein the multifilar conductor comprises a plurality of wires fabricated from an alloy containing at least one of cobalt, molybdenum, and chromium and provided with a coating of a biocompatible carbonaceous material that does not interact with the polyurethane.

3. The lead of Claim 2 wherein the multifilar conductor comprises a multifilar coiled conductor.

4. The lead of any one of the preceding claims wherein carbonaceous coating is 10 nm to 50 nm in thickness.

5. The lead of any one of the preceding claims wherein the biocompatible carbonaceous coating is of amorphous carbon.

6. The lead of any one of the preceding claims wherein the conductor comprises an elongated coil conductor.

7. Use of a metal, ceramic or carbon as a thin barrier film coating for an electrical conductor to prevent degradation of elastomer, for example, polyurethane insulation.
